# EUROPEAN PATENT SPECIFICATION

(11) **EP 4 419 644 B1**
(45) Date of publication and mention of the grant of the patent: **10.12.2025**
(21) Application number: 22789345.0
(22) Date of filing: 12.10.2022
(51) Int. Cl.: C12M 3/06, C12M 3/00, C12M 1/00, C12M 1/36

(54) **MICROINJECTION SYSTEM AND METHOD**
MIKROINJEKTIONSSYSTEM UND -VERFAHREN
SYSTÈME ET PROCÉDÉ DE MICRO-INJECTION

(30) Priority: 22.10.2021 NL 2029490
(43) Date of publication of application: 28.08.2024
(73) Proprietor: Life Science Methods B.V., 2333CH Leiden (NL)
(72) Inventor: DE SONNEVILLE, Jan, 2313 AW Leiden (NL)
(74) Representative: Nederlandsch Octrooibureau
(86) International application number: PCT/NL2022/050581
(87) International publication number: WO 2023/068924

(56) References cited:
- EP-B1- 2 991 706
- WO-A1-2008/034249
- WO-A1-2019/051072
- WO-A1-2019/211593
- CN-A- 108 070 618
- CN-U- 207 216 133
- JP-A- 2008 246 568
- US-A1- 2016 051 353
- US-A1- 2019 302 438
- ZHUANG SONGLIN ET AL: "Visual Detection and Two-Dimensional Rotation Control in Zebrafish Larva Heart Microinjection", IEEE/ASME TRANSACTIONS ON MECHATRONICS, IEEE SERVICE CENTER, PISCATAWAY, NJ, US, vol. 22, no. 5, 31 October 2017 (2017-10-31), pages 2003 - 2012, XP011671071, ISSN: 1083-4435, [retrieved on 20171012], DOI: 10.1109/TMECH.2017.2717860

## Description

### FIELD OF THE INVENTION

The present invention relates to a microinjection system, in particular to a microinjection system for microinjection of various types of biological samples. The invention also relates to a method of operating a microinjection system according to the invention. Furthermore, the invention relates to the uses of a microinjection system according to the invention.

### BACKGROUND OF THE INVENTION

Microinjection of a biological specimen is a direct and effective way to transfer external materials, such as cells, into said specimen. It has advantages over other methods of transferring materials into biological specimens, such as cells, because there is high delivery efficiency and low toxicity. Microinjection finds applications in many fields including genetic, reproductive and cancer research.

Traditionally, microinjection is done manually using a stereo microscope and a micromanipulator that uses micro screws to move a needle in three dimensions. However, this process has low efficiency and increases the chance of manual bias, errors and consequently a lower survival rate of the biological specimen. Additionally, the success rate of the injection is highly dependent on the professional skill and proficiency of the person performing the microinjection.

Automation of microinjection through microinjection robots has provided a more precise and effective process and reduces the chance of manual bias and errors. However, more complex injections are more difficult to automate, and so far efforts have only managed to solve issues in parts of the injection procedure. After all, there are many parts to the process that are essential to a successful microinjection.

Firstly, there is visualization of the sample to be injected. The sample to be injected needs to be clearly identifiable.

Secondly, there is the holding of the sample. In many cases the biological sample contains material that is alive, such as cells. The sample needs to be adjusted so it is in its proper position before injection and then needs to remain stable during the injection.

Thirdly, moving the needle into a suitable position through a motion platform needs to achieve accurate positioning for a successful injection into the sample. It is crucial that the needle is positioned in such a way that the sample is not damaged and that the injection can be achieved quickly.

Finally, there is the actual injection. The force exerted by the needle on the sample will inevitably cause a deformation of the sample. Therefore, the force of the injection needs to be accurately established in order to achieve a successful injection.

Numerous efforts have been made to address at least one, or more, of the above-mentioned steps in the microinjection process, by improving or changing different parts of a microinjection robot.

The company Eppendorf has commercialized an automated micromanipulator Injectman^{™} for microinjection that can perform predefined movements at the click of a button. However, the movement from sample to sample is not included, in addition, it lacks feedback during the injection process that is needed for more difficult types of injection.

A different proposed solution was specific sample holders that can be used to prepare samples at predefined locations in a predefined orientation. For example, using microfluidic solutions that use channels to guide samples, or cavities that are designed to fit samples perfectly. This can greatly simplify the injection process. Nevertheless, microfluidic positioning only works with a small subset of samples, and most cavities require manual manipulation of the samples to get them into the cavities. Additionally, it is also frequently required to manually adjust the samples within the cavities before injection can take place. Therefore, these procedures are slow and require extensive training before sample manipulation can be performed with a high efficiency.

Other approaches include the manual or (partly) automated procedure of picking a sample from a dish using a suction device, e.g. a blunt needle mounted on a micromanipulator. Followed by, for some applications, rotation of the sample or repositioning that can either be performed manually or automatically. Subsequently, injection can be performed manually or automatically using an injection needle mounted in a second micromanipulator. Picking a sample, and suctioning of a sample to prepare for injection is time-consuming, even when it is automated. Therefore, this type of approach is inefficient, which limits its use in experiments in which a lot of samples need to be injected in a small time window of biological development, or when a lot of samples need to be injected for, for example, screening.

Another issue that is often experienced is with microinjection of cells. For the application of microinjection of cells into a biological sample, such as tissue or an organism, cells are loaded into a needle. These cells often have the tendency to stick to each other, causing clogging of the needle. It takes time to form cell-cell contacts, and therefore, in these types of experiments, it is required to inject as many samples as possible before clogging of the needle takes place.

An example of prior art microinjection as described above can be found in the patent application US 2020/0308531 A, disclosing a robotic platform for high throughput injections into intact tissue. **In** an exemplary embodiment a robotic manipulator apparatus is provided and configured to hold and position a micropipette. Furthermore, the system comprises a microscope camera positioned to observe an injection site. A computing device receives image data from a microscope camera of the system, where the image data represents an image of a tissue sample. The computing device receives, via a user interface, an indication of a line traced by a user on the image of a tissue sample. **In** response, the computing device controls the robotic manipulator apparatus to move a tip of the micropipette along a path defined by the trajectory line. A pressure controller injects a gas into the micropipette to eject a substance out of the micropipette at one or more points along the path defined by the trajectory line.

The robotic manipulator as used in this disclosure positions the micropipette on a three axis manipulator as a result of which there is a limited degree of movement and orientation available for the micropipette.

Another example of a prior art microinjection system can be found in patent application US 2016/0051353 A1. This document discloses an automatic system for delivery of biologics into the organs of zebrafish larvae for use in *in vivo* screening. In the system the zebrafish larvae are immersed in a hydrogel in liquid state. A droplet of the hydrogel containing a single zebrafish larva is removed from the reservoir containing the hydrogel and placed in an array, which contains a cooler that cools the hydrogel so that it solidifies and immobilizes the larva. The larva can then be observed and injected with biologics.

The disadvantage of using such a system is that embedding specimen such as zebrafish larvae in a gel is slow and can affect zebrafish survival, especially when the larvae are only a couple of days old, e.g. two- or three-days post fertilisation.

US 2019/302438 A1 discloses a microscopic mirror imaging device and a system and method for calibrating a posture of a microneedle. The system includes the microscopic mirror imaging device, an inverted microscope system, a micromanipulation system, a needle holder rotational driver, a coronal plane angle driver, a sagittal plane angle driver, and a central processing device in which processing software is integrated. The inverted microscope system includes a video image acquirer and a manual or automatic focusing device.

In conclusion, there remains a need for efficient full automation of microinjection to increase the yield in microinjection applications.

### SUMMARY OF THE INVENTION

The present invention seeks to provide a microinjection system, in particular a microinjection system for microinjection of biological samples, such as microinjection of a liquid or suspension in biological samples, wherein the microinjection system enables high throughput of biological samples to be injected whilst maintaining high accuracy and reproducibility of the microinjections. The microinjection system provides improved motion control, sample illumination and improved imaging of biological samples.

According to the present invention, a microinjection system of the type defined above is provided, comprising:
a sample holder (2) configured to support a biological sample (S);
a first camera assembly (3) spaced apart from the sample holder (2); and
a needle holder (4) moveably arranged above the sample holder (2), wherein the needle holder (4) is configured to removably receive a hollow microneedle (5);
wherein the first camera assembly (3) is configured for imaging the biological sample (S) along an optical axis (O);
wherein the needle holder (4) and the sample holder (2) are spaced apart at a variable distance along the optical axis (O) for moving a tip part (6) of the microneedle (5) towards or away from the biological sample (S) when the microinjection system (1) is in use;
wherein the sample holder (2) is moveable in a plane (P) substantially perpendicular to the optical axis (O), and wherein
the needle holder (4) is rotationally arranged around the optical axis (O) for rotating the microneedle (5) around the optical axis (O).

A second aspect of the invention relates to a method of operating the microinjection system according to the invention comprising the steps of:
placing a microneedle (5) in the needle holder (4);
placing a biological sample (S) on the sample holder (2);
inserting, injecting a small volume, and retracting the tip part (6) in and from the biological sample (S) by one or more movements of
   a) rotating the needle holder (4) around the optical axis (O), and/or
   b) varying the distance between the needle holder (4) and the sample holder (2) along the optical axis (O), and/or
   c) moving the sample holder (2) in the plane (P) substantially perpendicular to the optical axis (O).

### BRIEF DESCRIPTION OF DRAWINGS

Figure 1 shows a schematic side view of a microinjection system according to a first embodiment of the present invention;
Figure 2 shows a schematic top view of a sample holder during an injection procedure according to an embodiment of the present invention;
Figure 3 shows an a schematic side view of an injection procedure according to an embodiment of the present invention;
Figure 4 shows a schematic side view of a first light source when in use according to an embodiment of the present invention;
Figure 5 shows a schematic side view of a second light source when in use according to an embodiment of the present invention;
Figure 6 shows a schematic side view a microinjection system according to a second embodiment of the present invention;
Figure 7 shows a schematic side view a microinjection system according to a third embodiment of the present invention;
Figure 8 shows a schematic side view a microinjection system according to a fourth embodiment of the present invention.

### DETAILED DESCRIPTION OF THE INVENTION

There is a need for overcoming barriers of access as experienced with microinjection systems of the prior art. Additionally, more extensive and time-consuming sample manipulation is required before a sample can be used. In some previous systems, sample manipulation is performed for a batch of samples, and often requires a specific sample holder, before injection can take place. In other previous systems samples have to be manually or automatically manipulated before each injection. Both types of manipulation increase the time before a batch of samples is injected. Using the precious time before injections can limit the throughput of experimentation, when injections need to take place in a certain time window of e.g. biological development.

There is a need for efficient, fully automated microinjection systems that can accurately and quickly inject multiple biological samples with a high throughput. The inventors have now created a microinjection system 1 that aims to solve at least part of this problem.

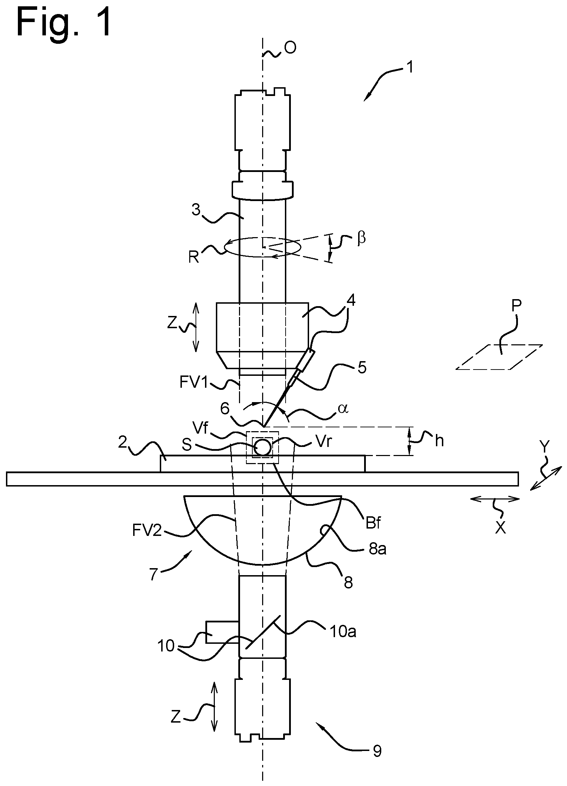

Referring to Figure 1, a microinjection system 1 for microinjection of biological samples is depicted according to an embodiment of the present invention. As shown, the microinjection system 1 comprises a sample holder 2 which is configured to support a biological sample S to be injected. A first camera assembly 3 is provided and spaced apart from the sample holder 2, wherein the first camera assembly 3 is configured for imaging the biological sample S along an optical axis O. For ease of understanding, in an embodiment the optical axis O may be considered to be vertical.

The microinjection system 1 further comprises a needle or pipette holder 4 which is moveably arranged above the sample holder 2, wherein the needle holder 4 is configured to removably receive a hollow microneedle or micropipette 5. Thus, the needle holder 4 allows the microneedle 5 to be disposed and renewed when required.

As depicted, the needle holder 4 and the sample holder 2 are spaced apart at a variable distance along the optical axis O for moving a tip part 6 of the microneedle 5 towards or away from sample holder 2, and thus the biological sample S, when the microinjection system 1 is in use. In particular, when in use, the variable distance between the needle holder 4 and the sample holder 2 allows a height "h" between the tip part 6 and the sample holder 2 to be varied. In the embodiment depicted, the needle holder 4 and the sample holder 2 are spaced apart at a variable distance along the optical axis O in the direction Z.

The sample holder 2, which supports the biological sample S when the microsystem 1 is in use, is moveable in a plane P substantially perpendicular to the optical axis O. The sample holder 2 is movable in an X and/or Y direction, wherein the X and Y direction are substantially perpendicular to the Z direction, i.e. substantially perpendicular to the optical axis O.

Furthermore, the needle holder 4 is rotationally arranged around the optical axis O for rotating the microneedle 5 around the optical axis O. As can be seen in Figure 1, the needle holder 4 is arranged to rotate around the optical axis O as indicated by the arrow direction R, i.e. in clockwise or counter clockwise direction.

The microinjection system 1 as described above allows for very accurate positioning of the tip part 6 of the microneedle 5 and the orientation thereof with respect to the biological sample S by one or more movements of a) rotating the needle holder 4, and thus the microneedle 5 around the optical axis O, and/or b) varying the distance between the needle holder 4 and the sample holder 2 along the optical axis O, and/or c) moving the sample holder 2 in the plane P substantially perpendicular to the optical axis O, e.g. in the X and/or Y directions as indicated.

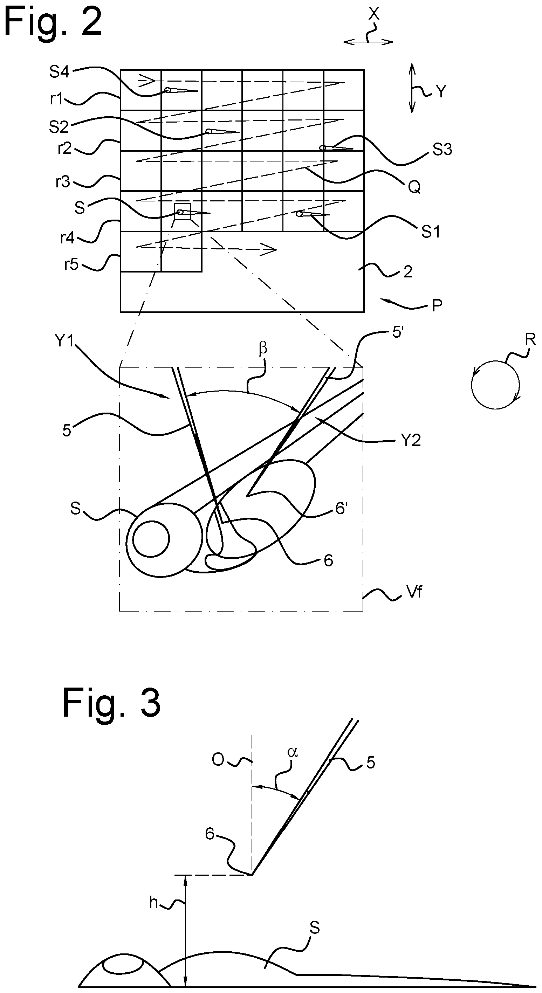

It is worth noting that for a plurality of biological samples S, S1, S2, S3, S4, see e.g. Figure 2, the microinjection system 1 allows injection of each of the biological samples in short succession through rapid changes and accurate adjustments according to the aforementioned movements a) to c). Most notably, the needle holder 4 allows each desired orientation of the microneedle 5 in the plane P and position of the tip part 6 to be achieved for optimal injection of each biological sample S, S1, S2, S3, S4 without the need to rotate the sample holder 2.

In an embodiment, the microinjection system 1 may comprise a control module (not shown) configured to rotate the needle holder 4 around the optical axis O, vary the distance between the needle holder 4 and the sample holder 2, and/or to move the sample holder 2 in the X and/or Y direction to allow the first camera assembly 3 to image the biological sample S and tip part 6 of the microneedle 5 for accurate and fast injection.

For achieving a further optimal orientation of the microneedle 5 with respect to the biological sample S, the needle holder 4 may be configured to hold the microneedle 5 at a pitch angle α, e.g. an adjustable pitch angle α, between 20° and 60° degrees with respect to the optical axis O. In this embodiment the needle holder 4 allows the microneedle 5 to engage the biological sample S in a skewed manner at the pitch angle α between 20° and 60°, or alternatively between 30° and 70° with respect to the plane P in which the sample holder 2 is configured to move. As a result, a wide variety of pitch directions of the microneedle 5 is available with respect to the optical axis O, so that the biological sample S and the tip part 6 can be imaged by the first camera assembly 3 from an optimal position.

In an embodiment, the aforementioned pitch angle α may be adjusted manually or even automatically, e.g. by means of the aforementioned control module. In this way the tip part 6 of a new microneedle 5 can be easily positioned as required, e.g. when a new microneedle 5 is mounted on the needle holder 4.

In the embodiment of Figure 1 it is further seen that the first camera assembly 3 may be configured to provide a focal volume Vf in which the biological sample S is imaged when in use, wherein the needle holder 4 is configured to provide a position space Vr of the tip part 6 of the microneedle 5. The position space Vr overlaps at least in part with the focal volume Vf. In this embodiment, the first camera assembly 3 is able to image the tip part 6 in the position space Vr when adjustments of the needle holder 4 have been completed, e.g. adjustments of the pitch angle α and/or rotation of the needle holder 4 around the optical axis O. Therefore, the position space Vr may be seen as a space in which the tip part 6 may be positioned or located when the microneedle 5 has been adjusted through rotation/orientation thereof with respect to the optical axis O.

To fully utilize the imaging capability of the first camera assembly 3, an embodiment is provided wherein the position space Vr is completely embedded or contained in the focal volume Vf. It is also advantageous to have an embodiment wherein the focal volume Vf and the position space Vr are substantially centred around the optical axis O for centred overlap between the focal volume and position space Vf, Vr.

As further depicted in Figure 1, in an embodiment the sample holder 2 may be arranged, when in use, above a bottom side Bf of the focal volume Vf to allow the biological sample S to be placed below the tip part 6 of the microneedle 5 but wherein the biological sample S remains within the focal volume Vf for being imaged by the first camera assembly 3.

In an embodiment, the first camera assembly 3 is configured to simultaneously focus on the biological sample S and the tip part 6 of the microneedle 5, thereby improving the imaging as to where the tip part 6 would come into contact with the biological sample S.

It was mentioned earlier that the needle holder 4 is configured to hold the microneedle 5 at a pitch angle α between 20° and 60° degrees with respect to the optical axis O for adjusting the pitch direction of the microneedle 5. To further increase the configurability of the orientation of the microneedle 5, an embodiment is provided wherein the needle holder 4 is rotatable around the optical axis O over a yaw angle range β of at least 30° degrees. Here, the yaw angle range β is measured around the optical axis O as indicated by the arrow direction R, e.g. clockwise counter clockwise, so wherein the yaw angle range β is measured in the plane P substantially perpendicular to the optical axis O. By allowing the needle holder 4 to rotate around the optical axis O over the at least 30° degrees is in most cases sufficient for most orientations of the biological sample S on the sample holder 2. In embodiments the yaw angle range β is at least 180° or at least 270° to allow for an even a larger number of orientations of the biological sample S on the sample holder 2.

From Figure 1 it can be inferred that the first camera assembly 3 is arranged above the sample holder 2. However, this is not necessary as the first camera assembly 3 may be arranged above or below the sample holder 2, thereby allowing for different imaging directions, e.g. from above or below, of the biological sample S and the tip part 6 of the microneedle 5. In case the first camera assembly 3 is arranged above the sample holder 2, an embodiment is conceivable wherein the needle holder 4 is rotatably mounted to the first camera assembly 3 and moves in unison therewith in the indicated Z direction for varying the distance between the needle holder 4 and the sample holder 2.

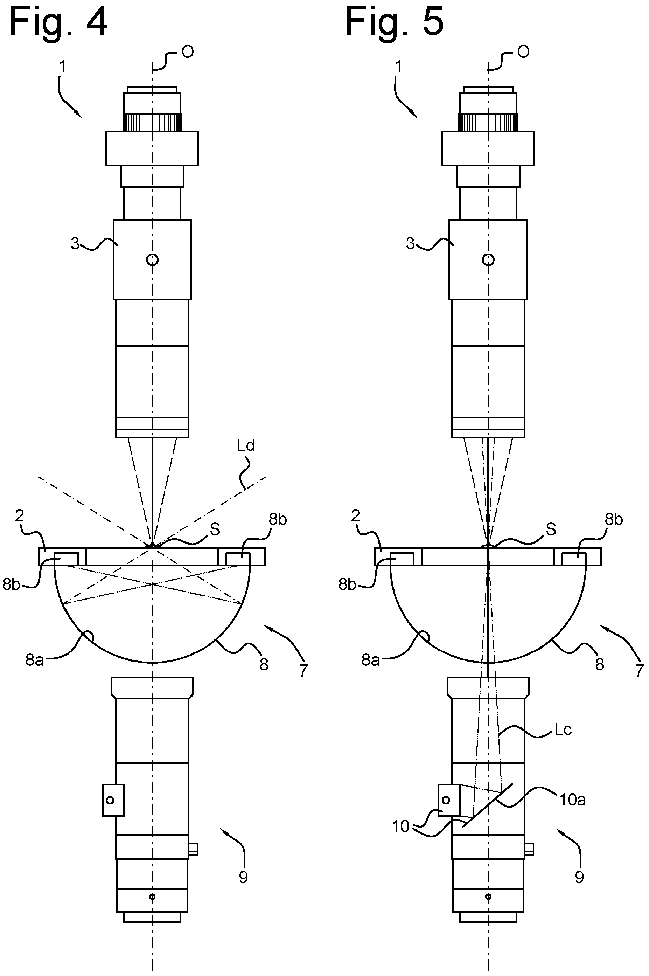

The microinjection system 1 of the present invention further improves illumination of the biological sample in various embodiments. As indicated in Figure 1, a high Numerical Aperture, NA, light source 7 is arranged under the sample holder 2 for illuminating the biological sample S from below. This high NA light source 7 increases image quality and reduces the dependence on surrounding light, e.g. room lighting. In some embodiments, one or more light sources may be provided and configured to emit light on the biological sample S at many different angles, thereby creating a high numerical aperture , NA, of illumination. To that end, an embodiment is conceivable wherein a plurality of light sources are arranged under the sample holder 2 in circular fashion to achieve a high NA light source 7. In another embodiment, the high numerical aperture light source 7 is a dome light source 8 which is configured to emit circumferentially distributed light around the optical axis O towards the biological sample S. Here, see e.g. Figure 4, the dome shaped light source 8 may comprise a reflective concave inner surface 8a and a circumferentially arranged light emitter 8b, wherein the concave inner surface 8a for reflecting emitted light Ld from the light emitter 8b in circumferentially distributed manner towards the biological sample S. In an embodiment, the dome light source 8 may be mounted to an underside of the sample holder 2 as shown.

In an embodiment, the first camera assembly 3 may be arranged above the sample holder 2 for imaging the biological sample S from above and wherein the microinjection system 1 further comprises a second camera assembly 9 arranged below the sample holder 2 spaced apart therefrom, wherein the second camera assembly 9 is configured for imaging the biological sample S from below along the optical axis O. The second camera assembly 9 may be very useful for obtaining an overview image of the biological sample S and optionally one or more further biological samples on the sample holder 2. That is, the second camera assembly 9 may be advantageous for locating the biological sample S and optionally one or more further biological samples and regions of interest for injection. The first camera assembly 3 is then utilized for detailed imaging of the biological sample S and the tip part 6 for the actual injection procedure.

In an embodiment, the first camera assembly 3 provides a first field of view FV1 which is smaller than a second field of view FV2 provided by the second camera assembly 7. In this embodiment the second camera assembly 9 has a second field of view FV2 that is larger than the first field of view FV1 of the first camera assembly 3. As such, the second camera assembly 9 allows for easy localisation of the biological sample S and optionally one or more further biological samples, as well as localisation of areas of interest to inject. The first camera assembly 3 allows for detailed imaging of a selected biological sample S and the tip part 6 during an injection procedure.

In an embodiment, the first camera assembly 3 provides a larger magnification factor than a magnification factor of the second camera assembly 9. Having a larger magnification factor for the first camera assembly 3 facilitates detailed, high resolution imaging of the biological sample S and tip part 6. The lower magnification factor for the second camera assembly 9 facilitates obtaining clear overview images of the biological sample S and optionally one or more further biological samples.

To further facilitate obtaining overview images, an embodiment may be considered wherein the second field of view FV2 is larger than a size of the biological sample S, so that the biological sample S and optionally one or more further biological samples can be imaged together with high image quality.

From Figure 1 it can be seen that the second field of view FV2 of the secondary camera assembly 9 overlaps the first field of view FV1 of the first camera assembly 3, thereby further ensuring that overview images can be obtained by the second camera assembly 9 and that detailed images can be obtained by the first camera assembly 3 without moving the sample holder 2.

It is worth noting that it will be convenient to arrange the first and second camera assembly 3, 9 on a shared optical axis, e.g. the optical axis O as depicted, but embodiments are conceivable wherein the first and second camera assembly 3, 9 have optical axes that are offset.

Further light sources may be added to the microinjection system 1 to improve overall imaging quality. For example, in an embodiment the second camera assembly 9 may comprise a low Numerical Aperture, NA, light source 10 configured to illuminate the biological sample S from below. This low NA light source 10 may be used to bring both the microneedle 5 and the biological sample S in focus at the same time and to predict where the tip part 6 would come into contact with the biological sample S, or to bring a larger part of the tip part 6 into focus to ease the ability to manually or automatically adjust the tip part 6 when a new microneedle 5 is mounted in the needle holder 4.

In an embodiment, the low NA light source 10 may be a coaxial light source. Such a coaxial light source may be generated using a collimated laser beam or using a telecentric bottom lens with a coaxial light input, for example. As shown in Figure 1 and Figure 5, the low numerical aperture light source 10, e.g. a coaxial light source, may emit light in a sideways fashion and through a half transparent mirror or prism 10a sending the emitted light Lc along the optical axis O towards the sample holder 2.

It is worth noting that the aforementioned high NA light source 7, e.g. dome light source 8, can be used in combination with the secondary camera assembly 9 and the low NA light source 10. For example, in an embodiment the high NA light source 7 may be arranged between the sample holder 2 and the second camera assembly 9. In case the high NA light source 7 is provided as a dome light source 8, then the dome light source 8 may comprise a through hole (not shown) centred on the optical axis O to allow the second camera assembly 9 to image the biological sample S and optionally the one or more further biological samples on the sample holder 2. The low NA light source 10 will be able to illuminate the biological sample S via the through hole in the dome light source 8.

As mentioned earlier, the microinjection system 1 of the present invention enables high throughput of biological samples to be injected. This is facilitated by the rotationally arranged needle holder 4 around the optical axis O, the movable sample holder 2 in the plane P perpendicular to the optical axis O, and the variable distance between the needle holder 4 and the sample holder 2. A method of operating the microinjection system 1 utilizing these movement capabilities can be described in further detail. For example, a method of operating the microinjection system 1 may be considered comprising the steps of:
placing a microneedle 5 in the needle holder 4;
placing a biological sample S on the sample holder 2;
inserting, injecting a small volume, and retracting the tip part 6 in and from the biological sample S by one or more movements of:
   a) rotating the needle holder 4 around the optical axis O, and/or
   b) varying the distance between the needle holder 4 and the sample holder 2 along the optical axis O, and/or
   c) moving the sample holder 2 in the plane P substantially perpendicular to the optical axis O, e.g. in the X and/or Y directions;

In movements under a), the microneedle 5 can be rotated in the yaw angle range β as mentioned earlier to obtain a desired yaw orientation of the microneedle 5 in the plane P without having to rotate the biological sample S in the plane P.

In movements under b), the distance between the needle holder 4 and the sample holder 2 can be varied along the optical axis O to adjust the distance between the tip part 6 and the sample holder 2 by changes of the height "h", e.g. insertion of the tip part 6 in the biological sample S and subsequently injecting of a volume therein, followed by retraction of the tip part 6 from the biological sample S.

In movements under c), the sample holder 2 may be moved in e.g. the X and/or Y direction to bring the biological sample S, or at least a required region thereof, under the tip part 6 of the microneedle 5.

It is worth nothing that movements under a) to c) may be performed in random sequence. For example, movement c) may be performed first, followed by movement a) and then followed by movement b). Allowing all movements a) to c) to be performed in any desired sequence allows for an efficient injection procedure with a minimum of errors and movements required, thereby increasing injection speed, and hence throughput of the microinjection system 1. It is even conceivable that movements a) to c) are performed simultaneously, thereby increasing injection speeds and throughput even further.

In an embodiment the method may comprise further a movement d) adjusting the pitch angle α with respect to the optical axis O, e.g. to reposition and/or recalibrate a newly supplied microneedle 5 in the needle holder 4 when a new injection procedure is started.

The method as outlined above can be readily automated, in part or completely, by the control module as suggested above, wherein the control module is arranged to automate the movements of a) to d) in any required sequence, possibly at least in part or completely simultaneously.

Figure 2 and 3 provide a general overview of how the microinjection system 1 could be used. It is seen that the sample holder 2 is depicted from above, i.e. in the plane P in which it is moveable. Movements in the X and/or Y direction under c) may allow an injection pattern Q to be followed whereby a plurality of biological samples S, S1, S3, S4 can be injected in sequence and in quick successions. In the embodiment of Figure 2, a sequential injection procedure in X-direction is depicted, i.e. successively injecting biological samples S, S1, S2, S3, S4 along a first row r1, followed by a second r2, and then further rows r3 to r5. Movements under a) allow the yaw angle β in the plane P to be adjusted to achieve a desired yaw orientation from Y1 to Y2 for example, i.e. to achieve a yaw orientation of the microneedle 5, 5' and tip part 6, 6' that allows for the most efficient injection of a particular region of a biological sample being processed.

In Figure 3 the movement under b) is further depicted, wherein the distance between the needle holder 4 and the sample holder 2 is varied to vary the height "h" between the tip part 6 and the biological sample S. Movements under d) show that the pitch angle α of the microneedle 5 can be adjusted with respect to the optical axis O, e.g. to reposition or recalibrate a newly mounted microneedle 5 in the needle holder 4.

For the embodiments wherein a second camera assembly 9 is provided, see e.g. Figure 1, the following further method of operating the microinjection system 1 may be considered also, comprising the more detailed steps of:
bringing the sample holder 2 into focus of the second camera assembly 9;
bringing a biological sample S on the sample holder 2 into focus of the second camera assembly 9;
moving the sample holder 2 to bring a region of the biological sample S below the tip part 6 of the microneedle 5;
rotating the needle holder 4 to rotate the microneedle 5 into a required injection direction;
bringing the region of the biological sample S into a focal volume Vf of the first camera assembly 3;
bringing the tip part 6 into contact with the biological sample S;
inserting the tip part 6 into the biological sample S;
injecting a small volume into the biological sample S; and
retracting the tip part 6 from the biological sample S.

This further method clearly shows the increased effectiveness of the first camera assembly 3 cooperating with the second camera assembly 9, wherein the second camera assembly 9 provides an overview image of a biological sample S on the sample holder 2, e.g. for locating a region of the biological sample S, and wherein the first camera assembly 3 provides a detailed image of the region of the biological sample S in the focal volume Vf of the first camera assembly 3. By using the first and second camera assemblies 3, 9, a highly automated yet accurate microinjection system 1 is provided whereby slow and error prone manual adjustments are avoided and where both localisation of biological samples S and the inj ection thereof can be effectively and efficiently performed by virtue of the second and first camera assembly 9, 3 respectively.

As mentioned above, from the embodiment in Figure 1 it is seen that the first camera assembly 3 is arranged above the sample holder 2. However, the first camera assembly 3 need not be arranged above the sample holder but may, in particular applications, be arranged below the sample holder 2 if so required. Choosing where to locate the first camera assembly 3 above or below the sample holder 2 may depend on whether the first camera assembly 3 is configured to function as a telecentric lens, a microscope or an inverted microscope.

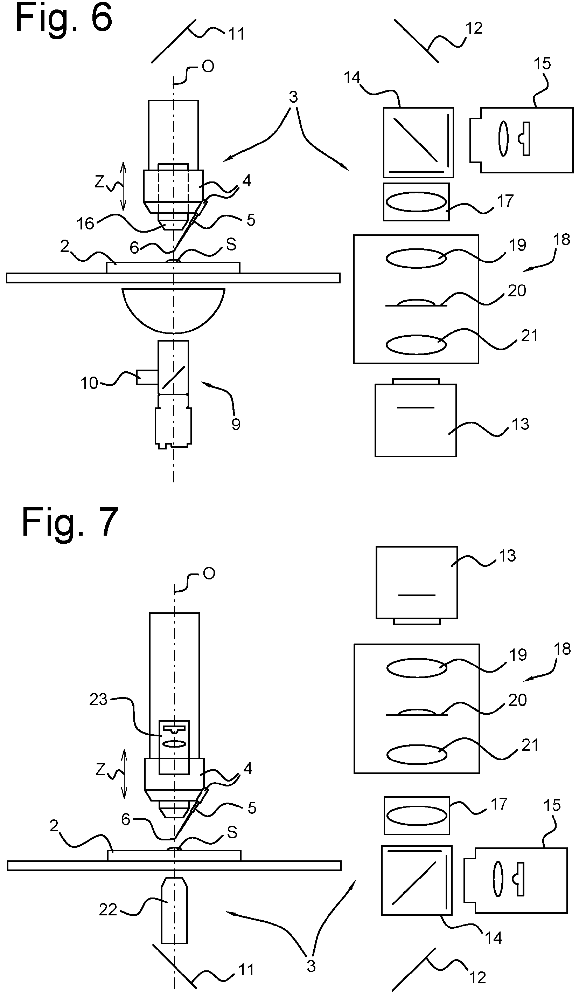

The embodiment shown in Figure 6 shows a schematic side view a microinjection system 1 according to an embodiment wherein the first camera assembly 3 is arranged above the sample holder 2 and configured as a microscope. Such a microscope is useful when smaller cells need to be injected, e.g. 300 micrometer in diameter, such as mouse embryos or even smaller. Smaller than 100 micrometer in diameter is also achievable, such as e.g. adherent cells.

In the depicted embodiment, the first camera assembly 3 may comprise a first mirror 11 and a second mirror 12 arranged above the sample holder 2, and a digital camera 13. Here, emitted light from the biological sample S travels in an upward direction along the optical axis O and is reflected by the first and second mirror 11, 12 downward towards the digital camera 13.

In an alternative embodiment, the first camera assembly 3 may comprise a first mirror 11, a fluorescent filter cube 14 and a cube light source 15 above the sample holder 2 and the aforementioned digital camera 13. When using the fluorescent filter cube 14, the cube light source 15 may be configured to illuminate the biological sample S for detecting fluorescently labelled sample structures of the biological sample S or a fluorescently labelled injection suspension.

In an embodiment, the first camera assembly 3 may comprise a first lens 16 which moves with the needle holder 4 in the Z direction. In an embodiment, the first lens 16 may be an air or water immersion objective lens, e.g. with a long working distance and based on infinity optics. Such a long working distance is advantageous to provide room for the microneedle 5 to be mounted in a pitch angle α which is as small as possible with respect to the optical axis O. The second mirror 12 or the fluorescent filter cube 14 are arranged to reflect light from the biological sample S towards the digital camera 13.

In various other embodiments, the first camera assembly 3 may comprise a tube lens 17 and a relay system 18 arranged between the second mirror 12 or the fluorescent filter cube 14 and the digital camera 13. The second mirror 12 and the fluorescent filter cube 14 are then arranged to reflect light from the biological sample S towards tube lens 14 , relay system 18 and to the digital camera. In an embodiment, the relay system 18 may comprise two relay lenses 19, 21 and a liquid lens 20 arranged there between. Using such a relay system 18 allows a focal plane from the first lens 16 to be shifted up and down.

As further depicted in Figure 6, the second camera assembly 9 may be provided to illuminate the biological sample 9 from below, e.g. by using the low numerical aperture light source 10.

In another embodiment, Figure 7 shows a microinjection system 1 similar to the embodiment of Figure 6 but wherein the first camera assembly 3 is arranged below the sample holder 2 and configured as an inverted microscope. In this embodiment the first camera assembly 3 may comprise the first mirror 11 and the second mirror 12 arranged below the sample holder 2, and the digital camera 13, thereby reflecting downward propagating light from the biological sample S from the first mirror 11 to the second mirror 12 upward to the digital camera 13.

In an alternative embodiment, the first camera assembly 3 may comprise the first mirror 11, the aforementioned fluorescent filter cube 14 and the cube light source 15 arranged below the sample holder 2, and the digital camera 13. Here, emitted light from the biological sample S travels in a downward direction along the optical axis O and is reflected by the first mirror 11 and the fluorescent filter cube 14, upward towards the digital camera 13. In an embodiment, the first camera assembly 3 may comprise a second lens 22 which is arranged above the first mirror 11 and below the sample holder 2. The microinjection system 1 may be further provided with a further light source 23 arranged for illuminating the biological sample S from above.

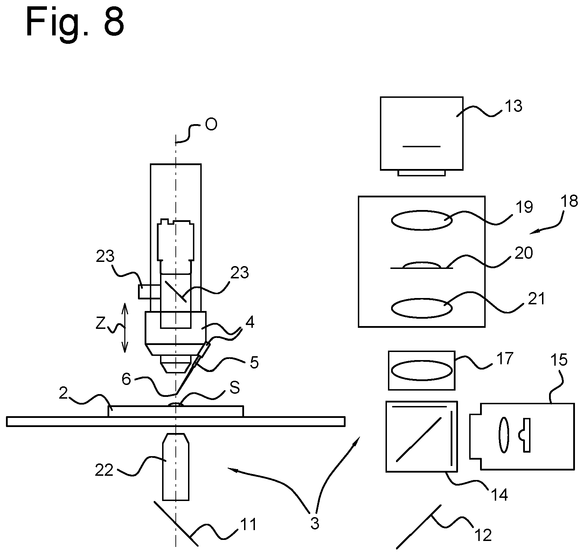

Figure 8 shows yet another embodiment of the microinjection system 1, and in particular the first camera assembly 3, wherein the first camera assembly 3 is arranged below the sample holder 2 and configured as an inverted microscope. In this embodiment, the first camera assembly 3 may comprise the first mirror 11 and the second mirror 12 arranged below the sample holder 2, and the digital camera 13. In an alternative embodiment, the first camera assembly 3 may comprise the first mirror 11, the fluorescent filter cube 14 and the cube light source 15 arranged below the sample holder 2, and the digital camera 13. Light from the biological sample S travels in a downward direction along the optical axis O and is reflected by the first mirror 11 and the second mirror 12 or fluorescent filter cube 14 upward towards the digital camera 13. In an embodiment, the first camera assembly 3 may comprise the second lens 22 which is arranged above the first mirror 11 and below the sample holder 2.

The microinjection system 1 may further comprise the further light source 23 arranged above the sample holder 2, wherein the further light source 23 may be a coaxial light source arranged to emit light in sideways fashion and through a half transparent mirror or prism downward to the biological sample S.

An aspect of the invention relates to the use of the microinjection system 1 according to the invention for the microinjection of small aquatic organisms such as *Danio rerio, Oryzias latipes, Xenopus laevis, Pimephales promelas* or *Xenopus tropicalis.* Preferably the aquatic organism is *Danio rerio* (zebrafish).

The aquatic organism is preferably at the embryonic stage of development, more preferably a zebrafish embryo. Zebrafish embryos have the advantage that they are transparent. Therefore, they are easy to use for assays and screens in which the internal organs of the organism have to be monitored.

Microinjection in small aquatic organisms is useful for toxicology assays, drug screenings or a combination thereof, as the compound of interest may be directly injected into the organism.

Microinjection of the compound into the organism presents several advantages over administering the compound through surrounding water so that the aquatic organism may absorb it.

Immersion exposure results in a relatively low uptake of the compound, which in turn may result in a false negative on the compound that is being tested. Additionally, the speed and amount of uptake is highly variable and is therefore not accurately reproducible. These problems can be circumvented by using microinjection, where uptake rate is not a factor.

Additionally, using microinjection, particularly pericardially or intraperitoneally, a compound can be rapidly distributed over the entire body of the organism and can yield relevant data on the effects of the compound on all internal organs of the aquatic organism, which can then be used as a model to predict how, for instance, a human subject would respond to the compound of interest.

Therefore, microinjection can be very useful for toxicology assays or drug screens, or combinations thereof.

In particular, when the system is used for toxicology assays or drug screens, the use of the microinjection system 1 according to the invention for taking samples from aquatic organisms, such as zebrafish, is contemplated. In toxicology assays or drugs screens it is very relevant to assess what is happening internally in the injected organism. It is essential to understand as well as monitor how the injected compound of interest affects the internal organs of the model aquatic organism, such as zebrafish, in particular zebrafish embryos. This can for instance be achieved by taking blood samples using microneedle 5, taking a biopsy using a biopsy needle in place of the microneedle 5 or taking a tissue sample using a microsurgical blade in place of the microneedle 5. For example, using a biopsy needle, a tissue sample can be extracted from a 3D cell culture or *ex vivo* tissue sample.

Additionally, the overall well-being of the aquatic organism may also be monitored by using for instance probes for electrophysiology instead of the microneedle 5. These probes may be used to obtain ECGs of the injected organism. Because of the separate moveability of both the needle holder 4 and the sample holder 2 of the microinjection system 1 according to the invention, it is now possible to place the probes, for instance 4 channel probes, in the exact same position on different aquatic organisms measured in sequence. This will give a more accurate representation of, on average, the organisms' overall well-being.

In an embodiment of the invention the injection that is injected into the aquatic organism, preferably zebrafish, more preferably zebrafish embryos, comprises human cells, wherein the human cells preferably originate from cell culture, fresh tissue material or are extracted from human blood.

Injecting zebrafish with human cells, in particular abnormal human cells such as tumour cells, can serve as a diagnostic modplaneel to establish what treatment a patient's disease may be responsive to. This would be an important step in generating personalised therapeutic approaches for patients.

The zebrafish embryo xenografts are much cheaper and easier to execute than the equivalent murine xenograft studies that are used and can be applied for a wide range of studies.

When zebrafish embryos are injected with human tumour cells, the cells rapidly proliferate, migrate, form masses and induce neo-angiogenesis, making zebrafish embryos excellent candidates for human tumour cell xenograft studies that look at treatment efficacy. Using these tumour cell xenografts it can, for instance, be determined whether the cells are radiosensitive or radioresistant or sensitive or resistant to chemotherapy.

Multiple cancer cell lines have already been tested in zebrafish embryos, including melanoma, glioma, adenocarcinoma, breast cancer, pancreatic cancer and prostate cancer cell lines.

Another use of microinjection of human cells is the injection of CAR (chimeric antigen receptor) T cells. In the last few years CAR T cell therapy has gained significant attention in the cancer treatment field. The cells have shown efficacy in the therapy of B cell malignancies and are now being explored for other malignancies. Zebrafish embryos that were previously injected with human cancer cells, as described here above, can be injected with CAR T cells after the cancer has proliferated in the embryo. It can then be studied whether the CAR T cells are effective against the particular tumour-type that the zebrafish embryo was exposed to. This is a very important preclinical model system for the development of CAR T cell therapies. The golden standard is currently murine studies, but using the microinjection system according to the invention could make these preclinical model studies much cheaper and much more efficient.

Another aspect of the invention relates to the use of the microinjection system 1 according to the invention for the microinjection of insects. Preferably, the insect is *Culex quinquefasciatus, Aedes aegypti* or *Lutzomyia longipalpis.*

In an embodiment the microinjection takes place during the embryonic stage of the insect.

In an embodiment the microinjection induces site-specific mutagenesis in the insect.

As is well known, insects can carry diseases that can infect other animals, including humans. In particular, mosquitoes are a source for interspecies transmission of disease. *Culex quinquefasciatus,* for instance, can carry diseases such as avian malaria, West Nile virus (WNV), Japanese encephalitis, Eastern equine encephalitis, lymphatic filariasis, and Saint Louis encephalitis. *Aedes agypti,* also known as the yellow fever mosquito, may carry dengue fever, chikungunya, Zika fever, Mayaro, yellow fever and other disease agents.

Sand flies, *Lutzomyia longipalpis,* are also a known carrier of disease, in particular leishmaniases.

To prevent spread of disease by these insects, research has been exploring site-specific mutagenesis in disease-carrying insects that addresses different aspects of the insect genome. The intended mutation can for instance lead to the insects becoming pathogen-resistant or creating sterile insects.

Microinj ection of insects can be improved with the microinjection system according to the invention, since one of the main challenges of microinjecting insects is orientating the insect embryo, larva or adult into a position in which injection can take place without risking that the injection will kill the specimen, while also ensuring that the injection occurs at a location that will lead to genome mutation. Due to the current system's unique moveable needle holder and sample holder, the specimen can now be moved into a suitable position without much effort and high precision.

Another aspect of the invention relates to the use of the microinjection system 1 according to the invention for the injection of cells, preferably human cells. In drug development, *in vitro* or *ex vivo* experiments using human cell lines, or human tissue, are often performed. The microinjection system according to the invention makes it much easier, more efficient and more accurate in injecting the compound of interest in the area of interest in the target cell(s). Using the microinjection system 1 according to the invention it is made possible to efficiently and accurately take, for instance, blood samples using microneedle 5, a biopsy using a biopsy needle in place of the microneedle 5 or a tissue sample using a microsurgical blade in place of the microneedle 5. For example, using a biopsy needle, a tissue sample can be extracted from a 3D cell culture or *ex vivo* tissue sample. Furthermore, it is also possible to fill the microneedle 5 with a cell suspension, and injecting new cells in the area of interest, for example in a tissue or hydrogel.

Additionally, by replacing the microneedle 5 with a micropipette filled with an electrolytic solution, patch clamp experiments may be performed to study the electrophysiology of the cells. Because of the free movement of the needle holder 4 and sample holder 2 of the microinjection system 1 according to the invention, it has now become easier to access a larger surface area of the cells/organs to be tested, which also allows for these cells/organs to be studied in 3D instead of 2D slices.

In view of the above, the present invention can now be summarised by the following embodiments:
Embodiment 1. A microinjection system (1) for microinjection of biological samples, comprising a sample holder (2) configured to support a biological sample (S); a first camera assembly (3) spaced apart from the sample holder (2); and a needle holder (4) moveably arranged above the sample holder (2), wherein the needle holder (4) is configured to removably receive a hollow microneedle (5); wherein the first camera assembly (3) is configured for imaging the biological sample (S) along an optical axis (O); wherein the needle holder (4) and the sample holder (2) are spaced apart at a variable distance along the optical axis (O) for moving a tip part (6) of the microneedle (5) towards or away from the biological sample (S) when the microinjection system (1) is in use; wherein the sample holder (2) is moveable in a plane (P) substantially perpendicular to the optical axis (O), and wherein the needle holder (4) is rotationally arranged around the optical axis (O) for rotating the microneedle (5) around the optical axis (O).
Embodiment 2. The microinjection system according to embodiment 1, wherein the first camera assembly (3) is arranged above or below the sample holder (2).
Embodiment 3. The microinjection system according to embodiment 1 or 2, wherein the needle holder (4) is configured to hold the microneedle (5) at a pitch angle (α) between 20° and 60° degrees with respect to the optical axis (O).
Embodiment 4. The microinjection system according to embodiment 1, 2 or 3, wherein the first camera assembly (3) is configured to provide a focal volume (Vf) in which the biological sample (S) is imaged during use, wherein the needle holder (4) is configured to provide a position space (Vr) of the tip part (6) of the microneedle (5), and wherein the position space (Vr) overlaps at least in part with the focal volume (Vf).
Embodiment 5. The microinjection system according to embodiment 4, wherein the first camera assembly (3) is configured to simultaneously focus on the biological sample (S) and the needle tip (6).
Embodiment 6. The microinjection system according to any one of embodiments 1-5, wherein the needle holder (4) is rotatable about the optical axis (O) over an yaw angle range (β) of at least 30° degrees.
Embodiment 7. The microinjection system according to any one of embodiments 1-6, wherein a high numerical aperture light source (7) is arranged under the sample holder (2) for illuminating the biological sample (S) from below.
Embodiment 8. The microinjection system according to any one of embodiments 1-7, wherein the first camera assembly (3) is arranged above the sample holder (2) for imaging the biological sample (S) from above and wherein the microinjection system (1) further comprises a second camera assembly (9) arranged below the sample holder (2) spaced apart therefrom, wherein the second camera assembly (9) is configured for imaging the biological sample (S) from below along the optical axis (O).
Embodiment 9. The microinjection system according to embodiment 8, wherein the first camera assembly (3) provides a first field of view (FV1) which is smaller than a second field of view (FV2) provided by the second camera assembly (7).
Embodiment 10. The microinjection system according to embodiment 8 or 9, wherein the first camera assembly (3) provides a larger magnification factor than a magnification factor of the second camera assembly (9).
Embodiment 11. The microinjection system according to embodiment 9, or embodiment 10 when depending from embodiment 9, wherein the second field of view (FV2) is larger than a size of the biological sample (S).
Embodiment 12. The microinjection system according to embodiment 9, or embodiment 10 or 11 when depending from embodiment 9, wherein the second field of view (FV2) overlaps with the first field of view FV1.
Embodiment 13. The microinjection system according to any one of embodiments 8-12, wherein the second camera assembly (9) comprises a low numerical aperture light source (10) configured to illuminate the biological sample (S) from below.
Embodiment 14. The microinjection system according to embodiment 7, or any one of embodiments 8-13 when depending from embodiment 7, wherein the high numerical aperture light source (7) is arranged between the sample holder (2) and the second camera assembly (9).
Embodiment 15. Method of operating the microinjection system (1) according to any one of embodiments 1-14 comprising the steps of:
   placing a microneedle (5) in the needle holder (4);
   placing a biological sample (S) on the sample holder (2);
   inserting, injecting a small volume, and retracting the tip part (6) in and from the biological sample (S) by one or more movements of
      a) rotating the needle holder (4) around the optical axis (O), and/or
      b) varying the distance between the needle holder (4) and the sample holder (2) along the optical axis (O), and/or
      c) moving the sample holder (2) in the plane (P) substantially perpendicular to the optical axis (O).

The present invention has been described above with reference to a number of exemplary embodiments as shown in the drawings and as outlined in the description.

## Claims

1. A microinjection system (1) for microinjection of biological samples, comprising
a sample holder (2) configured to support a biological sample (S);
a first camera assembly (3) spaced apart from the sample holder (2); and
a needle holder (4) moveably arranged above the sample holder (2), wherein the needle holder (4) is configured to removably receive a hollow microneedle (5);
wherein the first camera assembly (3) is configured for imaging the biological sample (S) along an optical axis (0);
wherein the needle holder (4) and the sample holder (2) are spaced apart at a variable distance along the optical axis (O) for moving a tip part (6) of the microneedle (5) towards or away from the biological sample (S) when the microinjection system (1) is in use;
wherein the sample holder (2) is moveable in a plane (P) substantially perpendicular to the optical axis (O), and wherein
the needle holder (4) is rotationally arranged around the optical axis (O) for rotating the microneedle (5) around the optical axis (O).

2. The microinjection system according to claim 1, wherein the first camera assembly (3) is arranged above or below the sample holder (2).

3. The microinjection system according to claim 1 or 2, wherein the needle holder (4) is configured to hold the microneedle (5) at a pitch angle (α) between 20° and 60° degrees with respect to the optical axis (O).

4. The microinjection system according to claim 1, 2 or 3, wherein the first camera assembly (3) is configured to provide a focal volume (Vf) in which the biological sample (S) is imaged during use, wherein the needle holder (4) is configured to provide a position space (Vr) of the tip part (6) of the microneedle (5), and wherein the position space (Vr) overlaps at least in part with the focal volume (Vf).

5. The microinjection system according to claim 4, wherein the first camera assembly (3) is configured to simultaneously focus on the biological sample (S) and the needle tip (6).

6. The microinjection system according to any one of claims 1-5, wherein the needle holder (4) is rotatable about the optical axis (O) over an yaw angle range (β) of at least 30° degrees.

7. The microinjection system according to any one of claims 1-6, wherein a high numerical aperture light source (7) is arranged under the sample holder (2) for illuminating the biological sample (S) from below.

8. The microinjection system according to any one of claims 1-7, wherein the first camera assembly (3) is arranged above the sample holder (2) for imaging the biological sample (S) from above and wherein the microinjection system (1) further comprises a second camera assembly (9) arranged below the sample holder (2) spaced apart therefrom, wherein the second camera assembly (9) is configured for imaging the biological sample (S) from below along the optical axis (O).

9. The microinjection system according to 8, wherein the first camera assembly (3) provides a first field of view (FV1) which is smaller than a second field of view (FV2) provided by the second camera assembly (7).

10. The microinjection system according to claim 8 or 9, wherein the first camera assembly (3) provides a larger magnification factor than a magnification factor of the second camera assembly (9).

11. The microinjection system according to claim 9, or claim 10 when depending from claim 9, wherein the second field of view (FV2) is larger than a size of the biological sample (S).

12. The microinjection system according to claim 9, or claim 10 or 11 when depending from claim 9, wherein the second field of view (FV2) overlaps with the first field of view (FV1).

13. The microinjection system according to any one of claims 8-12, wherein the second camera assembly (9) comprises a low numerical aperture light source (10) configured to illuminate the biological sample (S) from below.

14. The microinjection system according to claim 7, or any one of claims 8-13 when depending from claim 7, wherein the high numerical aperture light source (7) is arranged between the sample holder (2) and the second camera assembly (9).

15. Method of operating the microinjection system (1) according to any one of claims 1-14 comprising the steps of:
placing a microneedle (5) in the needle holder (4);
placing a biological sample (S) on the sample holder (2);
inserting, injecting a small volume, and retracting the tip part (6) in and from the biological sample (S) by one or more movements of
a) rotating the needle holder (4) around the optical axis (O), and/or
b) varying the distance between the needle holder (4) and the sample holder (2) along the optical axis (O), and/or
c) moving the sample holder (2) in the plane (P) substantially perpendicular to the optical axis (O).

## Patentansprüche

1. Mikroinjektionssystem (1) zur Mikroinjektion biologischer Proben, mit
einem Probenhalter (2), der zum Tragen einer biologischen Probe (S) ausgebildet ist;
einer ersten Kameraanordnung (3), die von dem Probenhalter (2) beabstandet ist; und
einem Nadelhalter (4), der beweglich über dem Probenhalter (2) angeordnet ist, wobei der Nadelhalter (4) konfiguriert ist, eine hohle Mikronadel (5) lösbar aufzunehmen;
wobei die erste Kameraeinheit (3) konfiguriert ist, die biologische Probe (S) entlang einer optischen Achse (0) abzubilden;
wobei der Nadelhalter (4) und der Probenhalter (2) entlang der optischen Achse (0) in einem variablen Abstand voneinander beabstandet sind, um einen Spitzenabschnitt (6) der Mikronadel (5) bei Verwendung des Mikroinjektionssystems (1) in Richtung auf die biologische Probe (S) zu oder von dieser weg zu bewegen;
wobei der Probenhalter (2) in einer zur optischen Achse (O) im Wesentlichen senkrechten Ebene (P) bewegbar ist, und wobei
der Nadelhalter (4) um die optische Achse (0) drehbar angeordnet ist, um die Mikronadel (5) um die optische Achse (O) zu drehen.

2. Mikroinjektionssystem nach Anspruch 1, wobei die erste Kameraanordnung (3) oberhalb oder unterhalb des Probenhalters (Z) angeordnet ist.

3. Mikroinjektionssystem nach Anspruch 1 oder 2, wobei der Nadelhalter (4) konfiguriert ist, die Mikronadel (5) in einem Teilungswinkel (α) zwischen 20° und 60° mit Bezug auf die optische Achse (O) zu halten.

4. Mikroinjektionssystem nach Anspruch 1, 2 oder 3, wobei die erste Kameraanordnung (3) so konfiguriert ist, dass sie ein Fokalvolumen (Vf) bereitstellt, in dem die biologische Probe (S) während des Gebrauchs abgebildet wird, wobei der Nadelhalter (4) so konfiguriert ist, dass er einen Positionsraum (Vr) für den Spitzenabschnitt (6) der Mikronadel (5) bereitstellt, und wobei der Positionsraum (Vr) zumindest teilweise mit dem Fokalvolumen (Vf) überlappt.

5. Mikroinjektionssystem gemäß Anspruch 4, wobei die erste Kameraanordnung (3) konfiguriert ist, gleichzeitig auf die biologische Probe (S) und die Nadelspitze (6) zu fokussieren.

6. Mikroinjektionssystem nach einem der Ansprüche 1 bis 5, wobei der Nadelhalter (4) um die optische Achse (O) über einen Gierwinkelbereich (ß) von mindestens 30° drehbar ist.

7. Mikroinjektionssystem nach einem der Ansprüche 1 bis 6, wobei eine Lichtquelle (7) mit hoher numerischer Apertur unterhalb des Probenhalters (2) angeordnet ist, um die biologische Probe (S) von unten zu beleuchten.

8. Mikroinjektionssystem nach einem der Ansprüche 1 bis 7, wobei die erste Kameraanordnung (3) oberhalb des Probenhalters (2) angeordnet ist, um die biologische Probe (S) von oben abzubilden, und wobei das Mikroinjektionssystem (1) ferner eine zweite Kameraanordnung (9) aufweist, die unterhalb des Probenhalters (2) in einem Abstand davon angeordnet ist, wobei die zweite Kameraanordnung (9) konfiguriert ist, die biologische Probe (S) von unten entlang der optischen Achse (O) abzubilden.

9. Mikroinjektionssystem gemäß 8, wobei die erste Kameraanordnung (3) ein erstes Sichtfeld (FV1) bereitstellt, das kleiner als ein von der zweiten Kameraanordnung (7) bereitgestelltes, zweites Sichtfeld (FV2) ist.

10. Mikroinjektionssystem nach Anspruch 8 oder 9, wobei die erste Kameraanordnung (3) einen Vergrößerungsfaktor bereitstellt, der größer als ein Vergrößerungsfaktor der zweiten Kameraanordnung (9) ist.

11. Mikroinjektionssystem nach Anspruch 9 oder Anspruch 10, wenn dieser von Anspruch 9 abhängig ist, wobei das zweite Sichtfeld (FV2) größer als eine Größe der biologischen Probe (S) ist.

12. Mikroinjektionssystem gemäß Anspruch 9 oder Anspruch 10 oder 11, wenn diese von Anspruch 9 abhängig sind, wobei das zweite Sichtfeld (FV2) das erste Sichtfeld (FV1) überlappt.

13. Mikroinjektionssystem nach einem der Ansprüche 8 bis 12, wobei die zweite Kameraeinheit (9) eine Lichtquelle (10) mit niedriger numerischer Apertur aufweist, die so konfiguriert ist, dass sie die biologische Probe (S) von unten beleuchtet.

14. Mikroinjektionssystem nach Anspruch 7 oder einem der Ansprüche 8 bis 13, wenn diese von Anspruch 7 abhängig sind, wobei die Lichtquelle (7) mit hoher numerischer Apertur zwischen dem Probenhalter (2) und der zweiten Kameraanordnung (9) angeordnet ist.

15. Verfahren zum Betätigen des Mikroinjektionssystems (1) nach einem der Ansprüche 1 bis 14, mit den folgenden Schritten:
Platzieren einer Mikronadel (5) in dem Nadelhalter (4);
Platzieren einer biologischen Probe (S) auf dem Probenhalter (Z);
Einsetzen, Injizieren eines kleinen Volumens und Zurückziehen des Spitzenabschnitts (6) in die und aus der biologischen Probe (S) durch eine oder mehrere Bewegungen, in Form von
a) Drehen des Nadelhalters (4) um die optische Achse (O) und/oder
b) Verändern des Abstands zwischen dem Nadelhalter (4) und dem Probenhalter (2) entlang der optischen Achse (O) und/oder
c) Bewegen des Probenhalters (2) in der Ebene (P) im Wesentlichen senkrecht zur optischen Achse (O).

## Revendications

1. - Système de micro-injection (1) pour la micro-injection d'échantillons biologiques, comprenant
un porte-échantillon (2) configuré pour supporter un échantillon biologique (S) ;
un premier ensemble caméra (3) espacé du porte-échantillon (2) ; et
un porte-aiguille (4) monté de manière mobile au-dessus du porte-échantillon (2), le porte-aiguille (4) étant configuré pour recevoir de manière amovible une micro-aiguille creuse (5) ;
dans lequel le premier ensemble caméra (3) est configuré pour imager l'échantillon biologique (S) le long d'un axe optique (O) ;
dans lequel le porte-aiguille (4) et le porte-échantillon (2) sont espacés à une distance variable le long de l'axe optique (O) pour rapprocher ou éloigner une partie formant pointe (6) de la micro-aiguille (5) vis-à-vis de l'échantillon biologique (S) lorsque le système de micro-injection (1) est en cours d'utilisation ;
dans lequel le porte-échantillon (2) est déplaçable dans un plan (P) sensiblement perpendiculaire à l'axe optique (O), et
dans lequel le porte-aiguille (4) est disposé apte à tourner autour de l'axe optique (O) pour faire tourner la micro-aiguille (5) autour de l'axe optique (O).

2. - Système de micro-injection selon la revendication 1, dans lequel le premier ensemble caméra (3) est disposé au-dessus ou au-dessous du porte-échantillon (2).

3. - Système de micro-injection selon la revendication 1 ou 2, dans lequel le porte-aiguille (4) est configuré pour maintenir la micro-aiguille (5) à un angle de tangage (α) entre 20° et 60° degrés par rapport à l'axe optique (O).

4. - Système de micro-injection selon l'une des revendications 1, 2 et 3, dans lequel le premier ensemble caméra (3) est configuré pour fournir un volume focal (Vf) dans lequel l'échantillon biologique (5) est imagé pendant l'utilisation, dans lequel le porte-aiguille (4) est configuré pour fournir un espace de position (Vr) de la partie formant pointe (6) de la micro-aiguille (5), et dans lequel l'espace de position (Vr) chevauche au moins en partie le volume focal (Vf).

5. - Système de micro-injection selon la revendication 4, dans lequel le premier ensemble caméra (3) est configuré pour se focaliser simultanément sur l'échantillon biologique (S) et la pointe d'aiguille (6).

6. - Système de micro-injection selon l'une quelconque des revendications 1 à 5, dans lequel le porte-aiguille (4) est apte à tourner autour de l'axe optique (O) sur une plage d'angles de lacet (β) d'au moins 30° degrés.

7. - Système de micro-injection selon l'une quelconque des revendications 1 à 6, dans lequel une source lumineuse à grande ouverture numérique (7) est disposée sous le porte-échantillon (2) pour éclairer l'échantillon biologique (S) par le dessous.

8. - Système de micro-injection selon l'une quelconque des revendications 1 à 7, dans lequel le premier ensemble caméra (3) est disposé au-dessus du porte-échantillon (2) pour imager l'échantillon biologique (S) par le dessus et dans lequel le système de micro-injection (1) comprend en outre un second ensemble caméra (9) disposé sous le porte-échantillon (2) et espacé de celui-ci, le second ensemble caméra (9) étant configuré pour imager l'échantillon biologique (S) par le dessous le long de l'axe optique (O).

9. - Système de micro-injection selon la revendication 8, dans lequel le premier ensemble caméra (3) fournit un premier champ de vision (FV1) qui est plus petit qu'un second champ de vision (FV2) fourni par le second ensemble caméra (7).

10. - Système de micro-injection selon la revendication 8 ou 9, dans lequel le premier ensemble caméra (3) fournit un facteur d'agrandissement plus important qu'un facteur d'agrandissement du second ensemble caméra (9).

11. - Système de micro-injection selon la revendication 9 ou la revendication 10 lorsqu'elle dépend de la revendication 9, dans lequel le second champ de vision (FV2) est plus grand qu'une taille de l'échantillon biologique (S).

12. - Système de micro-injection selon la revendication 9 ou la revendication 10 ou 11 lorsqu'elle dépend de la revendication 9, dans lequel le second champ de vision (FV2) chevauche le premier champ de vision (FV1).

13. - Système de micro-injection selon l'une quelconque des revendications 8 à 12, dans lequel le second ensemble caméra (9) comprend une source lumineuse à faible ouverture numérique (10) configurée pour éclairer l'échantillon biologique (S) par le dessous.

14. - Système de micro-injection selon la revendication 7 ou l'une quelconque des revendications 8 à 13 lorsqu'elles dépendent de la revendication 7, dans lequel la source lumineuse à grande ouverture numérique (7) est disposée entre le porte-échantillon (2) et le second ensemble de caméra (9).

15. - Procédé de fonctionnement du système de micro-injection (1) selon l'une quelconque des revendications 1 à 14, comprenant les étapes consistant à :
placer une micro-aiguille (5) dans le porte-aiguille (4) ;
placer un échantillon biologique (S) sur le porte-échantillon (2) ;
introduire, injecter un petit volume et rétracter la partie formant pointe (6) dans et à partir de l'échantillon biologique (S) par un ou plusieurs mouvements de
a) rotation du porte-aiguille (4) autour de l'axe optique (O), et/ou
b) variation de la distance entre le porte-aiguille (4) et le porte-échantillon (2) le long de l'axe optique (O), et/ou
c) déplacement du porte-échantillon (2) dans le plan (P) sensiblement perpendiculaire à l'axe optique (O).
